# EUROPEAN PATENT APPLICATION

(11) **EP 3 330 365 A1**
(43) Date of publication of application: **06.06.2018**
(21) Application number: 16201486.4
(22) Date of filing: 30.11.2016
(51) Int. Cl.: C12M 1/26, C12N 1/02, A23J 1/00, A23K 10/16, A23K 50/10, A23K 50/75, A23K 50/30, A23K 50/80, A23L 3/005, A23L 17/00, A23L 29/00, A23L 33/135, A23L 33/00, C12M 1/00, A23C 1/04, A21D 2/26, A21D 10/00, A21D 13/00

(54) **METHOD OF PROCESSING MICROALGAE BIOMASS**

(71) Applicant: Danmarks Tekniske Universitet, 2800 Kgs. Lyngby (DK)
(72) Inventor: Safafar, Hamed, 8270 Højbjerg (DK); Jacobsen, Charlotte, 2630 Tåstrup (DK); Reesbøll, Claus Asperud, 3450 Allerød (DK); Møller, Per, 4450 Jyderup (DK)
(74) Representative: Plougmann Vingtoft a/s

(57) **Abstract**

The present invention relates to a method of processing microalgae biomass, in particular to a method of processing microalgae biomass comprising the steps of membrane filtration, centrifugation and drying. The centrifugation is preferably performed with moderate centrifugal forces.

## Description

### Technical field of the invention

The present invention relates to processing of microalgae biomass. In particular, the present invention relates to processing of microalgae biomass by using a lower energy consumption while avoiding deterioration of bioactive compounds in the microalgae.

### Background of the invention

Microalgae are a diverse group of phototropic microorganism. Microalgae are considered an abundant, sustainable source of biochemical with high biomass productivity and the particular ability for conversion of carbon dioxide to oxygen and bioactive compounds. It has been estimated that microalgae produce more than 15000 metabolites. Microalgae can also grow on effluents from different sources and reduce their biological oxygen demand by conversions of nutrients such as ammonia and phosphorous to protein, lipids, vitamins, pigments and many other valuable compounds.

The microalgae ranges in size from a few micrometers (µm) to a few hundred micrometers. The term microalgae often includes cyanobacteria (blue green algae), diatoms and green algae. Microalgae biomass cultivation is receiving a great deal of attention as a potential source of bioactive compounds. Microalgae can be cultivated on low price growth media such as waste water and produce high amounts of protein, lipids, pigments and many other valuable compounds. Carbon dioxide (e.g. from combustion) can be used as a source of carbon for algal growth (1 kg of dry algal biomass requiring about 1.8 kg of CO2). It is believed that both productivity and growth rate of microalgae are much higher compared to terrestrial crops. Microalgae can produce high amounts of protein (up to 70%), lipids (up to 70%) and carotenoids (e.g. 20 mg/g dry weight). So microalgae biomass is a sustainable and environmental friendly product which can be used in different applications.

Today large scale cultivation of microalgae is being done in raceway open ponds or photo bio reactors which include flat panel airlift reactors, and tubular reactors. Concentration of microalgae biomass on harvest time depends on the growth condition and species and ranges from 100-600 mg/L. This low amount of dry matter as well as tough cell walls, high viscosity and heat sensitive bioactive compounds are main challenges in processing of microalgae.

Microalgae downstream processing includes a combination of two or more steps such as harvest, up concentration and drying, depending on the application of biomass.

The main harvesting procedures currently used include sedimentation, in gravity field, centrifugation, flotation and filtration. Centrifugation is the main method for up concentration, and several types of continuous centrifuges are used in industrial processes. Spray drying is the most important industrial method which is used for drying of microalgae biomass, but this method negatively affects some sensitive compounds such as carotenoids. The downstream processing of microalgae biomass is known as a major and costly component of production.

Most commercial producers of microalgae biomass use today a one step harvest and up-concentration method followed by direct spray drying of the obtained slurry. A one step method results in a high energy consumption as well as deterioration of bioactive compounds in the microalgae, e.g. the carotenoids,

Hence, an improved method of preparing microalgae would be advantageous, and in particular, a simple and effective method having a lower energy consumption while having no or low deterioration of the bioactive compounds would be advantageous.

### Summary of the invention

Thus, an object of the present invention relates to providing a method for processing of microalgae that is more efficient and economical than the technologies for processing microalgae known today. One advantage of the present invention is that it provides a method having a reduced energy consumption such that the production of microalgae for example is more economical. In addition, an object of the invention is to provide a method of processing microalgae with less negative effects on bioactive compounds. A further object is to provide a method, which provides a higher recovery of microalgae biomass with lower degradation and improved stability of the bioactive compounds present in the microalgae biomass. For example, the long chain fatty acids, lipids, proteins, pigments and other natural antioxidants in the microalgae biomass is maintained.

Thus, one aspect of the invention relates to a method for processing of microalgae biomass comprising the steps of:
a) harvesting microalgae by membrane filtration with a silicon carbide membrane, wherein a source of microalgae is passed through the silicon carbide membrane filter to obtain a retentate and a permeate
b) concentration by centrifugation of the retentate from step a) comprising a paste of microalgae biomass, and
c) drying the concentrated microalgae biomass from step b).

Another aspect of the present invention relates to a microalgae biomass obtainable by the method according to the present invention.

A further aspect of the present invention relates to the use of the microalgae biomass obtainable by the method according to the present invention in a feed or a food product.

### Brief description of the figures

Figure 1 shows the harvest performance (flux) for *Nannochloropsis salina* by using 300 mm silicon carbide membranes with different pore sizes (0.04 µm, 0.1 µm and 1.0 µm). Harvest experiments were done at constant pressure: 1.3±0.2 bar and temperature: 20±3°C.
Figure 2 shows harvest performance (flux) for *Nannochloropsis salina* by using 300 mm x 0.1 µm silicon carbide membranes at different temperatures. Harvest experiments were done at constant pressure 1.3±0.2 bar, and temperature 5±2°C, 15±3°C, and 30±5°C.
Figure 3 shows harvest performance (flux) for (a) *Nannochloropsis salina* and (b) *Chlorella vulgaris,* by using a silicon carbide membrane at different pressures. Harvest experiments were done at constant temperature 17±2°C, and different pressures (1±0.1 bar and 2±0.2 bar). The initial dry matter content for the two species under the experiment were 0.14 and 0.88 g/L for *Nannochloropsis salina* and *Chlorella vulgaris,* respectively.
Figure 4 shows microalgae biomass recovery for centrifugation with different relative centrifugal forces measured in "g". data was collected from up-concentrating several microalgae species including *Nannochloropsis salina, Chlorella vulgaris, Chlorella sorokiniana* and *Desmodesmus* species. Harvesting was with a silicon carbide membrane and concentrating was done at constant temperature 20 ±1°C, and for 10 minutes.
Figure 5 shows lipid and protein content (% dry-weight) variations in (a) *Nannochloropsis salina* and *Chlorella sorokiniana* paste prepared using different RCF's (1000-15000 g) under centrifugation. The microalgae was before up-concentration filtrated by using a silicon carbide membrane.
Figure 6 shows the fatty acid content composition in a *Nannochloropsis salina* paste prepared using different RCF's (1000-15000 g) under centrifugation. The microalgae was before up-concentration harvested by filtration with a silicon carbide membrane. Results were shown as % of total fatty acids.
Figure 7 shows the content of different pigments in a *Nannochloropsis salina* paste prepared using different RCF's (1000-15000 g) under centrifugation. The microalgae was before up-concentration filtrated by using a silicon carbide membrane. Results were shown as µg/g dry weight..
Figure 8 shows the relationship between the percentage of leakage and RCF values (1000-15000 g) for *Chlorella sorokiniana* and *Nannochloropsis salina.* The microalgae was before up-concentration harvested by filtration with a silicon carbide membrane. Results are shown in % dry-weight.
Figure 9 shows the dynamic (absolute) viscosity of microalgae paste prepared by centrifugation at various RCF's. Data was shown for (a) *Nannochloropsis salina* and *Chlorella sorokiniana.* The microalgae was before up-concentration harvested by filtration with a silicon carbide membrane. Centrifugation was done at constant temperature 20±1°C for 10 minutes.
Figure 10 shows the dry matter content in microalgae pastes prepared by centrifugation at different RCF's. data was collected from up-concentration of three microalgae species: *Nannochloropsis salina, Chlorella vulgaris* and *Chlorella sorokiniana.* The microalgae was before up-concentration harvested by filtration with a silicon carbide membrane. Centrifugation was done at constant temperature 20±1°C for 10 minutes.
Figure 11 shows the effect of heat treatment (75°C for 15 seconds) on the fatty acid composition of harvested biomass slurries from *Nannochloropsis salina.*
Figure 12 shows the effect of heat treatment (75°C for 15 seconds) on the pigment composition of harvested biomass slurries from *Nannochloropsis salina.*

The present invention will now be described in more detail in the following.

### Detailed description of the invention

### Definitions

Prior to discussing the present invention in further details, the following terms and conventions will first be defined:
The term microns refer in the context of the present invention to "micrometer".

In the context of the present invention, mentioned percentages are by weight (weight/weight) percentages unless otherwise stated.

The term "and/or" used in the context of "X and/or Y" should be interpreted as "X", or "Y", or "X and Y".

### Microalgae

The term microalgae refers in the context of the present invention any type of microalgae and therefore includes cyanobacteria (blue green algae), diatoms and green algae.

The microalgae used for microalgae downstream processing according to the present invention may be any kind of microalgae. It is not of relevance for the method which microalgae is processed. Thus the microalge may for example be any one of *Nannochloropsis sp., Chlorella sp., Monodopsis sp., Arthrospira* sp., *Phaeodatylum sp., Dunaliella sp.,* and *Desmodesmus sp.* or a combination thereof. For example the microalgae species may be any one of *Nannochloropsis salina, Arthrospira* sp., *Chlorella vulgaris, Chlorella sorokiniana, Chlorella minutissima, Chlorella pyrenoidosa, Monodopsis subterranea, Phaeodatylum tricornutom, Dunaliella salina,* and *Desmodesmus* sp., or a combination thereof. These types of microalgae is only to be seen as examples and not a limitation of the invention. Different microalgae have different content of bioactive compounds. For example, *Chlorella pyrenoidosa* has a high protein content, while *Nannochloropsis salina* has a high content of the long chain fatty acid, eicosapentaenoic acid. *Chlorella vulgaris* comprises on the contrary a high amount of carotenoids.

The cell size of microalgae varies from a few microns to a few hundred microns. An example of a small microalgae is *Arthrospira species,* which is only a few microns large. *Nannochloropsis species* is an example of larger microalgae. Biochemical compositions of microalgae highly varies and depends on several factors such as light and temperature. In general, microalgae include 8-70% protein, 4-70% carbohydrates and 2-50% lipids. Microalgae biomass also includes bioactive compounds such as carotenoids, pigments, antioxidants, fatty acids, vitamins and coenzyme Q10.

Microalgae biomass cultivation is receiving a great deal of attention as a potential source of bioactive compounds.

The method according to the present invention for downstream processing of microalgae biomass has the advantage that its energy consumption is lower, compared with processing methods known today, which results in improved economy in connection with production of microalgae biomass.

Microalgae can be cultivated on low price growth media such as wastewater.

### Harvest of microalgae

The term "harvest", "harvesting" and variations hereof, refers to increasing the biomass density of the microalgae. Harvesting microalgae may also be defined as increasing dry matter in a microalgae suspension, i.e. a reduction of fluid volume and/or increase in biomass density. After harvesting according to the present invention, a paste or slurry containing microalgae biomass is obtained.

Harvest of microalgae is the first step of the downstream process of microalgae and plays a major role in the energy consumption of the whole process and quality of the biomass.

The harvest step comprises harvesting of microalgae by microfiltration with a silicon carbide membrane, wherein a source of microalgae is passed through the silicon carbide membrane filter.

The term "source of microalgae" will in the context of the present invention relate to a suspension of microalgae after cultivation. The suspension of microalgae will typically be microalgae suspended in a growth medium. Under the harvest step the growth medium is filtrated away. The growth medium may for example be industrial process water. However, the growth medium may be any medium suitable for growth of microalgae and is not limited to process water. Thus, in an embodiment of the invention, the source of microalgae is a suspension of microalgae. In a preferred embodiment, the source of microalgae is a suspension of microalgae in growth medium.

The term "membrane filtration" and "microfiltration" refer in the present invention to the same step, namely filtration with a silicon carbide membrane.

The concentration of microalgae biomass in growth medium is typically very low (0.1 to 5 g/L).

In the present invention, the harvest of microalgae biomass by membrane filtration with a silicon carbide membrane results in separation of microalgae biomass without any adverse effects on the quality and with minimum possible required energy.

### Membrane filtration

The membrane filtration used for the harvest step according to the present invention will retain the microalgae biomass in a retentate after passing through the membrane, while the growth medium will pass the membrane and therefore be present in the permeate.

The membrane used in the membrane filtration according to the present invention is a silicon carbide membrane.

A silicon carbide membrane is a ceramic membrane.

In an embodiment of the invention, the pore size of the silicon carbide membrane is in the range of 0.05 to 0.5µm, such as in the range of 0.08 to 0.2 µm, preferably 0.1µm.

In a further embodiment of the invention, the pressure during membrane filtration is not more than 3 bar, such as in the range of 1 to 3 bar, preferably 1-2 bar.

The temperature used for the membrane filtration may in principle be any temperature. However, it has been shown that the harvest performance is increased with temperatures in the range of 15-20°C±5°C. Thus, in an embodiment of the invention the temperature under membrane filtration is in the range of 10-25°C, preferably 15-20°C.

The membrane filtration may be carried out by using dead-end membrane filtration or cross-flow membrane filtration. Dead-end membrane filtration is typically used for filtration of larger microalgae such as for example *Arthrospira* sp. and provides higher concentrations of the paste. Cross-flow membrane filtration is typically used for membrane filtration of small size microalgae.

In the method according to the invention, the microalgae suspended in growth medium flows tangentially across the membrane and a biomass paste is obtained (retentate).

In an embodiment of the invention, the harvested microalgae (retentate) may be recycled to the feed tank, so that the concentration of microalgae in the feed increases to a constant level (final concentration). Thus, an embodiment of the inventionrelates to that the method further comprises recirculation of the retentate from step a) to the source of microalgae and repeating step a) at least two times before concentration in step b).

An advantage of harvesting microalgae by using membrane filtration is that the salt content is being washed out during membrane filtration into the permeate. This is an advantage compared to other harvest methods, since those methods will require an additional washing procedure to remove salt from the biomass.

The permeability of salt and salt complexes is not the same for different membranes, however, the inventors of the present invention have surprisingly found out that salt is efficiently washed out in the permeate when using a silicon carbide membrane.

### Flocculants

In an embodiment of the invention, a flocculant is added to the source of microalgae, i.e. the suspension of microalgae in growth medium, before harvesting.

The flocculant may in an embodiment of the invention be a chemical flocculant, such as aluminium oxide, iron chloride (iron (III) chloride, cationic polymers, chitosan and surface active agents.

Addition of a flocculant before harvesting may ease the harvest step such that the separation of microalgae biomass and growth medium is more easy.

The flocculant may also be a bio-flocculant, for example bacterias. Bacterias that may be used as bio-flocculants are *Paenibacillus* sp., *Burkholderia cepaciahas.*

### Centrifugation

After harvest of the microalgae biomass, the microalgae biomass (the retentate from the filtration step) is centrifuged in order to concentrate the microalgae biomass. During the centrifugation, solids are sedimented and liquids are filtrated away.

During the downstream processing of microalgae, the microalgae cells are being affected by the hydrodynamic forces from mechanical processes such as pumping, mixing or centrifugation. These external forces could cause cell wall rupture when too high. Microalgae cell wall strength is not the same, so some species are more sensitive to such external stresses. Smaller forces cause cell death without membrane fracture. Cell rupture has both the active and adverse consequences, depending on the aim of the process. When the microalgae biomass is supposed to be used in a food or feed, e.g. fish feed, the quality of the bioactive compounds (nutrients) is of great importance and it is thus necessary that the cell wall of the microalgae has not ruptured. Rupture of cell wall can lead to leakage of lipid and a fatty acids. It is an object of the present invention to avoid rupture of the cell wall or at least minimize the rupture.

The centrifugal force, which may also be termed as centrifugal power, influences the separation efficiency of the centrifugation. The higher the centrifugal force is, the higher is the efficiency. For example, centrifugation of microalgae suspended in growth medium will in average result in obtaining 95% microalgae by centrifugation using a centrifugal force of 13.000g, while the efficiency is 60% at 6.000g, and 40% at 1.300g. However, the recovery of microalgae is dependend on the type of microalgae used.

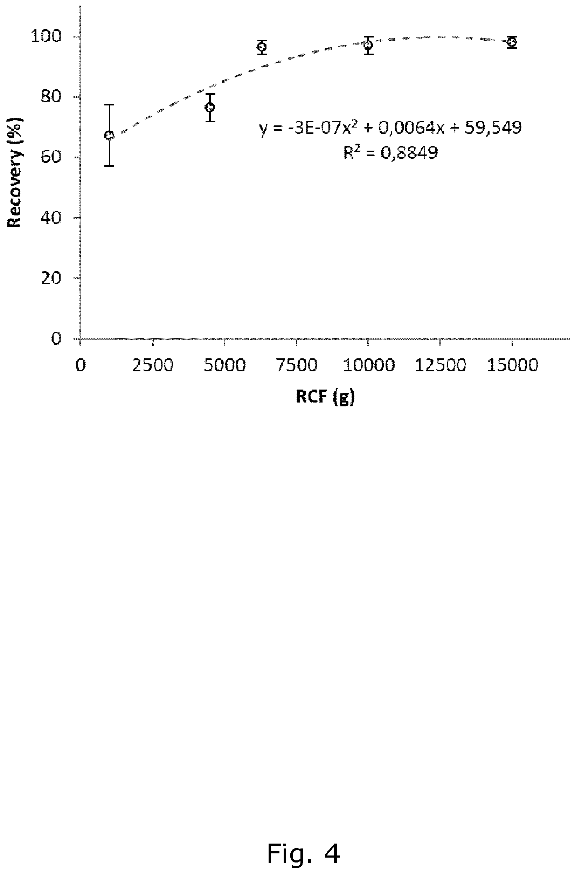

In the present invention, where centrifugation is performed on harvested microalgae (microalgae paste obtained from membrane filtration with a silicon carbide membrane), the separation efficiency of the centrifuge is more than 99% when using a centrifugal force of 6000g or above. When centrifugation is made with a centrifugal force of 4500g, the recovery efficiency is about 75%, while the recovery efficiency of microalgae is about 65% using a centrifugal force of 1000g, see figure 4.

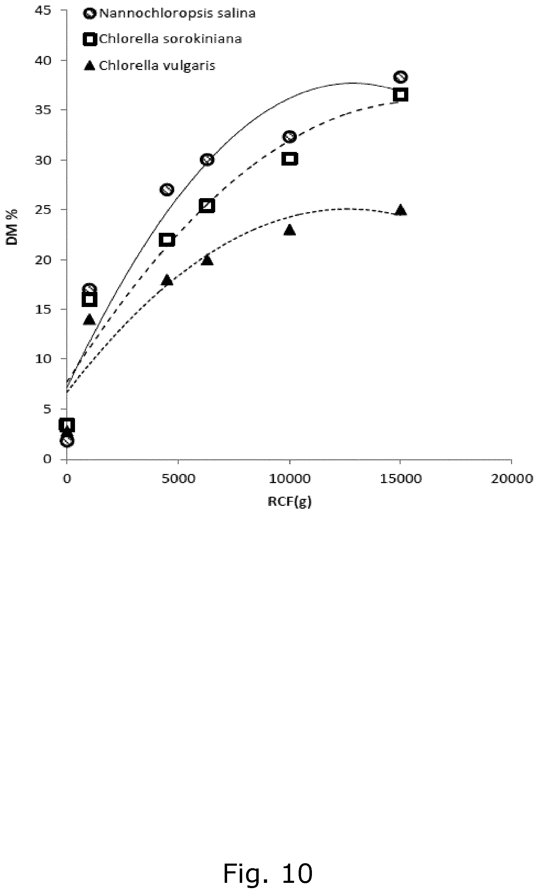

Figure 10 shows the dry matter content (%DM) for three microalgae, *Nannochloropsis salina, Chlorella sorokiniana* and *Chlorella vulgaris* after harvesting with a silicon carbide membrane and centrifugation with different centrifugal forces. Figure 10 shows that the dry matter content is increased dependent on the centrifugal force used under centrifugation.

Hence, it is an important aspect of the present invention, that both membrane filtration and centrifugation is used in order to process the microalgae biomass. If only centrifugation is used, a much higher centrifugal force is needed in order to have a high efficiency of microalgae biomass. In addition, if only membrane filtration is used in processing microalgae and no centrifugation step is added, the microalgae paste (retentate) obtained from membrane filtration with the silicon carbide membrane will have very high moisture content and therefore require a very high energy consumption when drying. It is an object of the present invention to reduce the consumption of energy.

In an embodiment of the invention, the centrifugal force is at least 4000g, such as at least 5000g, preferably at least 5500g, even more preferably at least 6000g. In a preferred embodiment of the invention, the centrifugal force is at least 5500g.

However, the packing factor "Ø" is defined as the volumetric proportion of solid to the liquid in the paste. Both average cell size and cell shape affect the packing factor. For *Chlorella vulgaris,* which has a comparative large cell size (7-9µm) the maximum packing factor was reported to be Ø=0.637 for a dry matter concentration of 159.25 g/L. On the contrary, microalgae such as *Chlorella vulgaris* can produce extracellular polymeric substances under stress condition and overcome the mechanical stresses such as hydrodynamic forces. These compounds bond water and hence, the separation of the bonded water would require very high centrifugal forces . Thus, increasing the centrifugal forces in processing *Chlorella vulgaris* would not alter the dry matter concentration in the biomass. For *Chlorella sorokiniana* and *Nannochloropsis salina* higher concentrations of dry matter was obtained than for *Chlorella vulgaris* at elevated centrifugal forces. Increasing biomass concentration, however, do not increase the pumping cost since the pumping efficiency increases with higher RCF values.

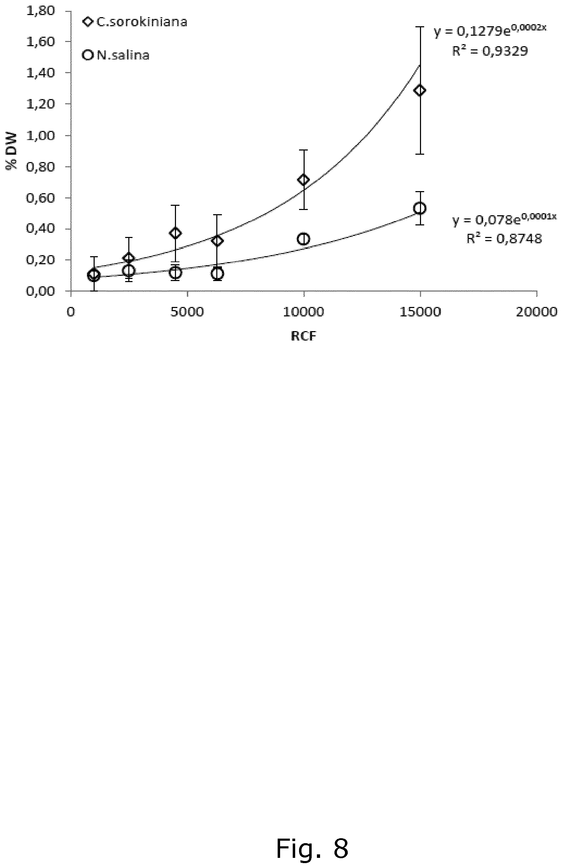

The inventors of the present invention has also found out that centrifugation using high centrifugal forces results in deterioration of the bioactive compounds in the microalgae. This is due to rupture of the cell wall of the microalgae. The combination of these external forces causes rupture or leakage of the lipids to the water phase. For microalgae with larger cell size and thinner cell walls, this phenomenon could result in a loss of 40% of the lipid. Lipid content (both in membrane and intercellular lipids), cell wall strength, amounts of damaged cells because of previous mechanical stresses and cell size are critical factors that could affect the degree of leakage in microalgae. Figure 8 shows that the higher the centrifugal force is under centrifugation, the higher is the leakage of lipids.

The inventors of the present invention has found out that when increasing the relative centrifugal force (RCF) under centrifugation, the cell wall is ruptured which results in a decrease in lipid content and fatty acid content. When microalgae biomass is used for food or feed it is important to maintain bioactive compounds, and in particular lipids and fatty acids.

Therefore, the present inventors found out that the harvested microalgae could be centrifuged using centrifugal forces of about 4000 g to 10000 g, such as 5000-8000 g preferably 5500-8000 g, even more preferably 6000 g to 7000 g to avoid cell rupture. The centrifugation is preferably done in a bowl centrifuge, such as a tubular bowl centrifuge.

The polyunsaturated fatty acids, such as eicosapentaenoic acid (EPA), belong to the group of fatty acids called omega-3 fatty acids. The animal or human body itself cannot generate these fatty acids. Instead, they must be obtained through the diet. The animal or human can convert the short-chain version of omega-3 fatty acids, alpha-linolenic acid (ALA), which is present in high amount in plant oils, to a long-chain version (EPA, DHA) to make use of it., but this conversion of ALA to EPA and DHA does not happen efficiently. Thus, a supply is of EPA and DHA is needed in the animal and human from other sources than ALA in plant oils.

EPA has beneficial anti-inflammatory effects and beneficial effects in preventing cardiovascular diseases.

With the method according to the present invention, microalgae biomass may be produced having a high content of fatty acids, including EPA, and lipids and avoids unnecessary loss of lipids and fatty acids.

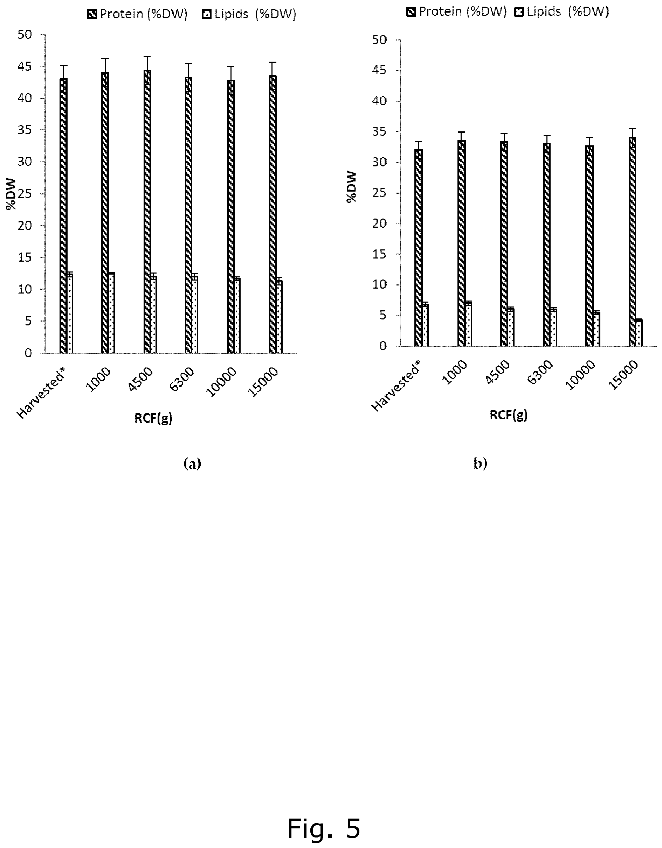

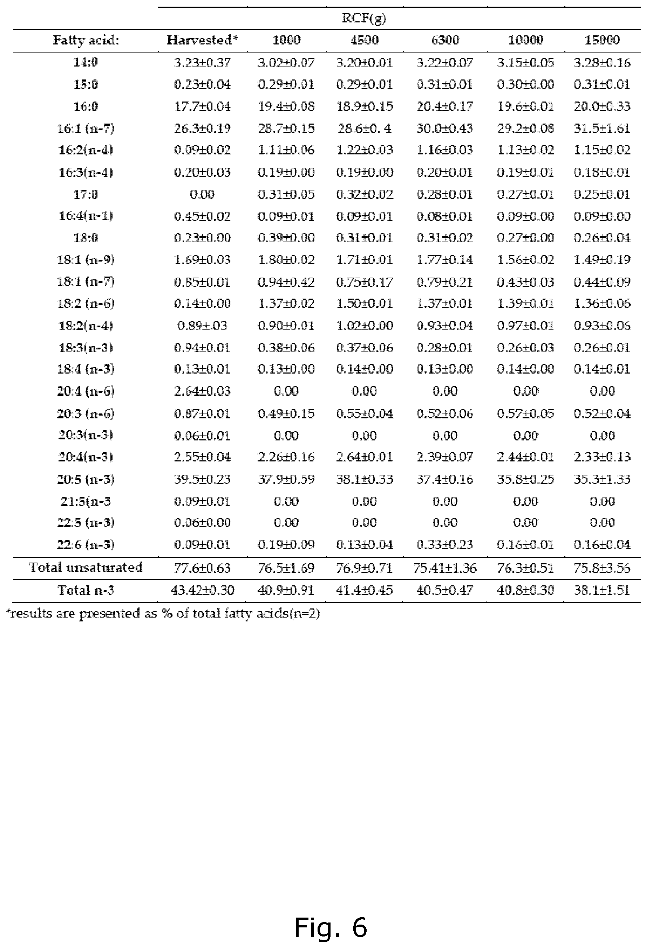

From figure 5 and 6, it can be seen that the lipid content and fatty acid content decreases in microalgae biomass when the relative centrifugal force (RCF) during centrifugation is increased. This is due to rupture of the cell wall of the microalgae when too high a centrifugal force is used under centrifugation. In particularly, it is shown in figure 6 that when the RCF value increase, the contents of the polyunsaturated fatty acids decrease. Microalgae membrane lipids comprise polyunsaturated fatty acids such as eicosapentaenoic acid (EPA). EPA has very beneficial effects to health and rupture of cell wall results in loss of these compounds.

Furthermore, cell wall rupture due to high centrifugal forces under centrifugation can also be seen since the content of pigments is increased as centrifugal forces is increases. This is due to cell rupture, which results in better extractability of the pigments.

The inventors of the present invention has found out that when up concentrating the harvested biomass by centrifugation with use of a moderate centrifugation force, the obtained microalgae biomass has a low moisture content and surprisingly no any rupture of the cell wall.

Thus, in a preferred embodiment of the invention, the centrifugal force under centrifugation is in the range of 4000 g to 10000 g., preferably, the centrifugal force is in the range of 5000 g to 9000 g, such as in the range of 5500 to 8000 g, preferably in the range of 6000 to 7500 g, even more preferably in the range of 6000 g to 7000 g.

In the method according to the present invention the centrifugal force during centrifugation is for example in the range of from 6000g to 8000g. This range is advantageous since it results in high yield of microalgae without deterioration of the microalgae.

### Drying

Drying is the last step of the microalgae downstream processing. In the drying step the moisture content is further decreased.

The drying step is an important step of the downstream processing of microalgae biomass since a microalgae biomass paste with a high moisture content is not suitable for applications such as food and feed where a dry biomass is required, because a dry microalgae biomass has an increased shelf life which is required for feed and food.

Drying of microalgae is required to facilitate the transportation, extend the shelf life and storage ability and to ease the applications for food and feed formulations.

Drying is the last step in method of processing microalgae. Drying requires large amounts of energy and involves irreversible deteriorations to the product. In order to reduce the drying time and thus reduce energy consumption, it is an object of the invention to have a microalgae biomass with a low moisture content before drying. This is obtained with the combination of harvesting by membrane filtration and up-concentrating the microalgae biomass by centrifugation according to the present invention.

After the centrifugation step, a microalgae biomass paste is obtained with a solid content of 20-40% dry matter weight. However, after the drying step the moisture content of the microalgae biomass is reduced to less than 10% dry matter weight.

In addition, storage, transportation and application of dried microalgae biomass is more economical than wet biomass paste.

The drying step according to the present invention may be any one of the following drying methods; sun drying, fluidised bed drying, spray drying, drum drying, incinator drying, cabinet drying, flash drying, spin flash drying and freeze drying. In a preferred embodiment of the present invention, the drying step is by use of a spin flash dryer.

Flash drying is a drying method where feed is injected into the bottom of a drying tube, in which a stream of hot gas is mixed with the biomass. The biomass hereby loses the moisture rapidly while moved by the drying stream. The gas is usually air.

Preferably, the dryer is a spin flash dryer having scraping paddles which moves close to the drying chamber walls (2mm) providing that the feed introduced to the drying chamber is immediately scraped from the walls and fluidised in the stream of the air. Drying occurs in seconds and the desity of smaller particles reduces so that they can be moved with the stream of air to the outlet. Larger particles return continuously to the scapping area and can move to the outlet and being distributed to the smaller sizes of biomass.

Sun (solar) drying includes two types of drying, indirect drying and direct drying. The indirect drying method uses hot air heated up by solar energy as drying agent. This prevents the products from overheating. Overheating may occur in the direct drying method.

Drum drying include a moving cylinder with facilities for deposition of the feed in thin layers on the hot surface of the drum and scraping blades to remove the dried layers. The design of the drum dryer to be used in the present invention may comprise a single, two or more scraping blades.

Cabinet drying is another example of drying to be used in the present invention. Cabinet drying can be categorized as direct air (cross flow) or vacuum cabinet drying.

In fluidised bed drying, pressurized drying stream passes through the particulate medium, giving properties of normal fluids, such as the ability to free-flow under gravity to the particulate medium.

Spray drying is a preferred method of drying the microalgae biomass slurry according to the present invention. Spray drying is a preferred method when the product is aiming for consumption, i.e. to be used in a feed or food. The spray drying method is continuous and can handle large amounts of biomass, while having a low drying time, as low as a few seconds. The microalga biomass slurry is sprayed in the drying chamber through a stream of hot gas. The dried product is afterwards removed from the bottom of the dryer. The spray drying method is very efficient, but due to the high pressure induced by the atomization process and very high temperature (over 200°C), and some bioactive compounds may therefore be lost.

Freeze drying is another example of a drying method used in the present invention. Freeze drying is a high quality, efficient and expensive drying technique, where the biomass is being dehydrated under high vacuum, This method is typically used in high-value products. However, freeze-drying is quite expensive. It has also been shown by the present inventors that at the freezing step before freeze-drying, ice crystals grow inside the cell, which results in cell rupture,. On the contrary, endogenous enzymes remain intact, which is a disadvantage.

### Microalgae biomass

The microalgae biomass obtained by the method according to the present invention (harvest, up concentrating and drying) has a high content of protein, a moderate amount of lipids, and elevated levels of bioactive compounds such as carotenoids, tocopherols, essential amino acids and long chain polyunsaturated fatty acids. The method according to the present invention of preparing a microalgae biomass has the advantage that cell rupture of the microalgae is avoided or at least minimized. In addition, the present method provides a microalgae biomass product with a high content of carotenoids and lipids, such as a high content of polyunsaturated fatty acids, which is desired in the final product.

In an aspect of the invention, the protein content in the microalgae biomass remain unaffected.

In an embodiment of the invention, the amount of protein in the obtained microalgae biomass is at least 25% by weight of dry matter, such as at least 30% by weight of dry matter, preferably at least 32% by weight of dry matter. The amount of protein may for example be from 25-70% by weight based on dry matter. In a preferred embodiment of the invention, the amount of protein in the microalgae biomass obtained by the method according to the present invention is 30-60% by weight of dry matter.

The amount of lipids in the in the obtained microalgae biomass is at least 7% by weight of dry matter, preferably at least 10% by weight based on dry matter. In a further embodiment of the invention, the amount of lipids in the obtained microalgae biomass is in the range of 5-60% by weight based on dry matter, such as from 10-55% by weight based on dry matter. In a particularly preferred embodiment of the invention, the amount of lipid in the obtained microalgae biomass obtained by the present invention is in the range of 40-50% by weight based on dry matter. A biomass with this high amount of lipids will have a high EPA content and be suitable for dietary supplements.

### Heat treatment

In an embodiment of the invention, the microalgae biomass obtained by harvesting, up concentrating and drying according to the present invention may furthermore be subjected to heat treatment. Heat treatment of the microalgae biomass provides inactivation of enzymes in the microalgae, which extend the shelf life of dried microalgae biomass.

Microalgae contain enzymes such as endogenous enzymes, lipase and lipoxygenase, which could degrade the cellular lipids, especially in the wet microalgae biomass. The free fatty acids in a microalgae biomass could increase very fast due to the presence of enzymes such as lipase in the microalgae cell. Hence, inactivation of enzymes could extend the shelf life of lipid containing microalgae biomass. Inactivation could be by heat treatment of the microalgae biomass.

On the contrary, some compounds in the microalgae is heat sensitive, such as carotenoids and highly unsaturated fatty acids. Thus, a gentle heat treatment such as pasteurisation which inactivate the enzymes but have less adverse effects on the heat sensible bioactive compounds is preferred.

Thus, in a preferred embodiment of the invention, the method according to the invention further comprises a step of pasteurization the harvested microalgae before centrifugation. Thus, the method further comprises a step of pasteurization of the retentate obtained in step a) of the present invention before concentration in step b).

The pasteurization may for example be done at a temperature of 60-120°C for 5-30 seconds, such as 60-100°C for 5-30 seconds, preferably 65-90°C for 10-25 seconds. Most preferably pasteurization is at a temperature of 75°C for 15 seconds. This pasteurization parameters showed the least deterioration of carotenoids, pigments and fatty acids and eligibility of the enzyme inactivation.

In another embodiment of the invention, the heat treatment is made during the drying step, such as using a heat dryer. A heat dryer could for example be a spray dryer, drum dryer or cabinet dryer. The temperatures in theses typers of dryers could be adjusted to 60-120°. A gentle drying method, such as freeze drying, would not heat treat the microalgae biomass, and therefore pasteurization could be performed when using freeze drying for drying the microalgae biomass.

### Use

Microalgae can be used in different applications. In general, applications of microalgae can be categorized into two broad areas; fuel and non-fuel. Fuel applications include biodiesel, methane, ethanol, and hydrogen. Non-fuel applications are nutraceuticals, food and feed, pharmaceuticals, wastewater treatment and pollution control, cosmetics, and chemicals such as biopolymer and lubricants.

The method according to the present invention, is particularly suitable for non-fuel applications and in particular for food and feed products, since the method results in a high yield of biomass without destroying the bioactive compounds, i.e. the microalgae biomass obtained by the present invention is highly suitable for food and feed products.

In an aspect of the invention, the microalgae biomass obtainable by the method according to the invention is used in a feed.

In an embodiment of the invention, the feed is a feed for pigs, cows, poultry or fish. In a preferred embodiment the microalgae biomass obtainable according to the present invention is uses in a fish feed.

In a further aspect of the invention, the microalgae biomass obtainable by the method according to the invention is used in a food product.

In an embodiment of the invention, the food product is selected from the group of dough, bread, buns, cake, pasta, cookies and energy bars.

It should be noted that embodiments and features described in the context of one of the aspects of the present invention also apply to the other aspects of the invention.

All patent and non-patent references cited in the present application, are hereby incorporated by reference in their entirety.

The invention will now be described in further details in the following non-limiting examples.

### Examples

### Methods and analyses used

Protein determination: The protein content in the microalgae samples was estimated using a modified micro Biuret method (Safafar, H., Z. Hass, M., Møller, P., L. Holdt, S., & Jacobsen, C. (2016). High-EPA Biomass from Nannochloropsis salina Cultivated in a Flat-Panel Photo-Bioreactor on a Process Water-Enriched Growth Medium. Marine Drugs.)

The amino acid composition was analysed by LC-MS, using EZ:faast™ amino acid analysis (Phenomenec Inc. CA, USA) (Safafar, H., Z. Hass, M., Møller, P., L. Holdt, S., & Jacobsen, C. (2016). High-EPA Biomass from Nannochloropsis salina Cultivated in a Flat-Panel Photo-Bioreactor on a Process Water-Enriched Growth Medium. Marine Drugs).

Lipids were extracted with a mixture of chloroform, methanol, and water, as described by Bligh and Dyer (1959).

Fatty acid profile was analysed by gas liquid chromatography according to the AOCS official method (Firestone 2009).

Analysis of tocopherols and tocotrienols was done by using LC-FLD Corporation, Massachusetts, USA, using a mixture of isopropanol and heptane (0.5:99.5) as the mobile phase (Firestone 2009).

Pigments extraction and analysis were done by the method described by: Safafar, H., van Wagenen, J. M., Møller, P., & Jacobsen, C. (2015). Carotenoids, Phenolic Compounds and Tocopherols Contribute to the Antioxidative Properties of Some Microalgae Species Grown on Industrial Wastewater. Marine Drugs, 13(12), 7339-7356. doi:10.3390/md13127069.

Measurement of moisture was done by an AD 4714A moisture analyser (A&D Company, Tokyo, Japan).

Viscosity measurement was done by senior rotary rheometer (Mars II, Haake, Germany).

Heat treatment experiments were done in borosilicate glass tubes (25-250 mL), in a temperature controlled water bath. Samples were cooled immediately after heating in a cold water container.

### Example 2 - Harvest - test of different pore size of membrane

In order to evaluate the effect of membrane pore size on harvest performance, a culture of *Nannochloropsis salina* was harvested by using silicon carbide membranes having different pore sizes.

The harvest of *Nannochloropsis salina* was done in batch mode by a silicon carbide membrane (SiC) microfiltration from Liqtech A/S, Denmark. A pilot scale filtration unit was designed and constructed for laboratory scale trials. The silicon carbide membranes (diameter=25mm and length=305 mm, where the channels in the tube has a diameter of 3 mm) with different pore sizes (0.04, 0.1 and 1 µm) have been evaluated. The harvest experiments were done at constant pressure 1.3±3°C bar and temperature 20±3°C.

The capacity of the microfiltration unit used was 20 L/h, and the unit was used for filtration test of different microalgae species. 10-20 L if growth medium (feed) was utilized for each species. For marine species, salt content was also evaluated in both permeate and harvested phases.

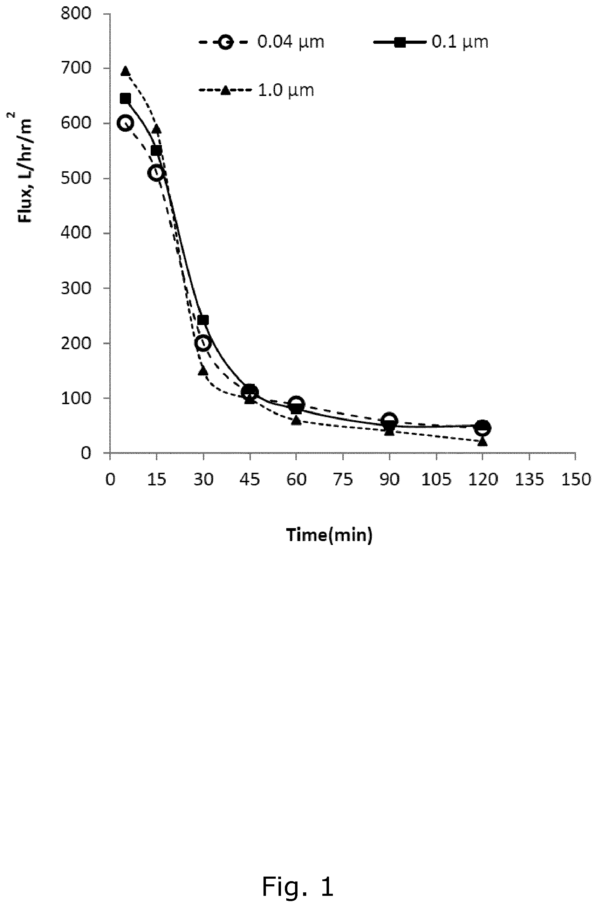

The result of the experiment is shown in figure 1.

As shown in figure 1, harvest by 1.0 µm started at higher fluxes but declined to a low flux after 120 minutes. The flux for membranes with a smaller pore sizes reached the steady state after 60 minutes, with no difference between 0.04 and 0.1 µm membranes. Due to the higher pressure drop, energy consumption increases when the pore size decreases, so a membrane with 0.1 µm pore size would be a better candidate for the harvest of microalgae with the small size range (2-4µm).

The membrane having a pore size of 1.0µm destroys the cell wall of the microalgae and is therefore not suitable. Thus, a pore size around 0.1µm is preferred.

### Example 3 - Harvest - effect of the temperature

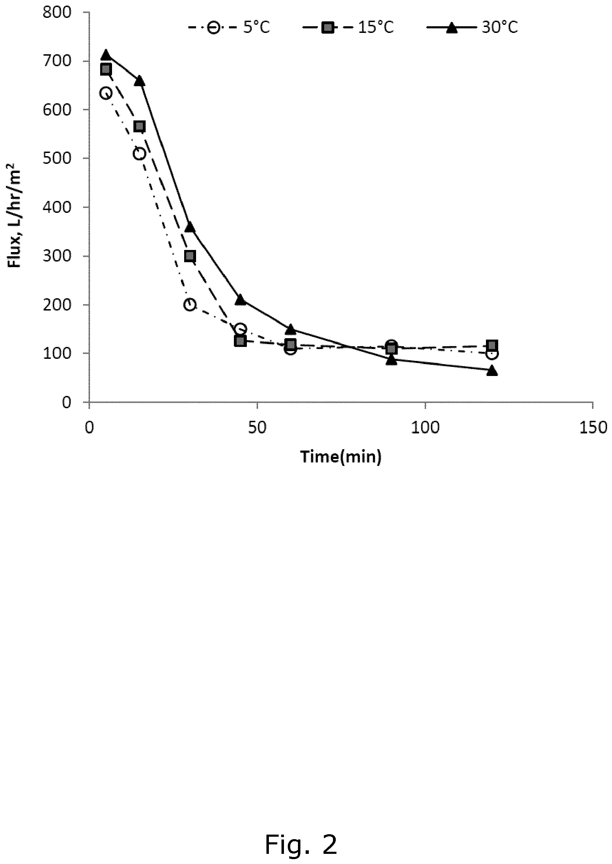

Effect of the temperature on the flux for a Nannochloropsis salina culture was evaluated. The harvest of Nannochloropsis salina was performed as in example 2, but the pore size was maintained at 0.1 µm for all samples and instead the temperature was varied. The temperature was 5°C, 15°C and 30°C respectively. The result of the experiment is shown in figure 2.

As shown in figure 2, the harvest performance (flux) started at higher rates at higher temperatures, but declined faster for the 30±5°C experiment, compared to lower temperatures. This finding can be explained by the fact that microalgae cell wall is more rigid at low temperature. On the other hand, the viscosity of fluids increases when the temperature decreases which negatively affects the filtration performance, so filtration in a range of 15-20°C will be the optimum temperature for harvest of intact, non-ruptured microalgae with a rigid cell wall.

### Example 4 - harvest - effect of pressure

The effect of the transmembrane pressure on harvest performance has been evaluated. Harvest experiments were done at constant temperature, 17±2°C, and with different pressure 1±0.1 bar and 2±0.4 bar. Pressure was measured and controlled at the filter hose outlet stream. The initial concentrations of dry matter were 0.14 and 0.88 g/L for *Nannochloropsis salina* and *Chlorella vulgaris,* respectively.

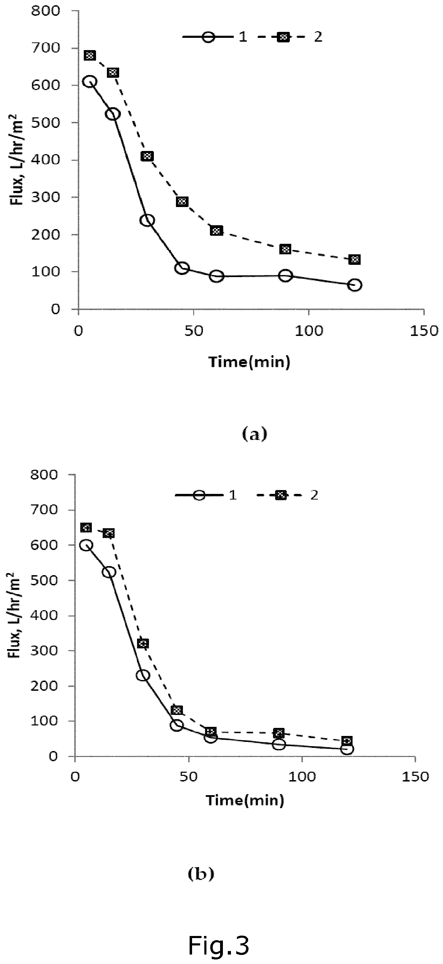

The result of the experiment is shown in figure 3. (a) shows the harvest performance (flux) of *Nannochloropsis salina* and (b) shows the harvest performance of *Chlorella vulgaris.*

As shown in figure 3, the flux increased when the pressure increased. However, in a biological fluid like a microalgae culture, there are many different factors which can affect the flux during the time and change the filtration efficiency at various pressures. The so-called transmembrane pressure for a silicon carbide membrane should be less than 3 bars. Higher pressures showed an adverse effect on the long term harvest efficiency but changed the filtration behaviour of the filter, especially for low pore size membranes, e.g. 0.04-0.1µm membranes (not shown here).

As shown in figure 3, filtration of *Nannochlorosis salina* at 2 bar pressure increased the flux during the harvest for 120 minutes. Final concentration ratio (C) was also apparently higher (24.3%) when compared to the lower pressure harvested sample (18%).

For the *Chlorella vulgaris* sample, surprisingly no significant differences were observed in the harvest efficiency.

### Example 5 - Effect of recovery using different centrifugal forces in the centrifugation step

Harvest by membrane filtration with a silicon carbide membrane and up-concentrating by centrifugation was tested on *Nannochloropsis salina, Chlorella vulgaris, Chlorella sorokiniana* and *Desmodesmus sp.* where the harvest parameters was the maintained the same, but the centrifugal force under centrifugation was varied. The centrifugal forces varied from 1000-15.000g. The microalgae cultures were before up-concentration by centrifugation harvested by using a silicon carbide membrane (300 mm x 0.1µm) at room temperature at 1.2±0.2 bar.

The centrifugation was done at a constant temperature 20±1°C for 10 minutes in 500 ml polyethene centrifuge tubes by using a bench scale batch centrifuge (Sorvall RC 6 Plus form Thermo scientific, Wlatham, MA, USA).

The biomass recovery was measured for the samples where the centrifugal force has been varied, see figure 4.

Biomass recovery depends on several factors such as cell size, growth stage, the percentage of damaged/dead cells and the initial concentration of the culture. As shown in figure 4, the values of relative centrifugal forces (RCF) of more than 6000 g were estimated to be higher than 99.2%. Deviations in the biomass recovery at lower RCF was high. The recovery for small size and damaged microalgae cells was lower than the others. When RCF values greater than 6000 g were applied, regardless of the parameters mentioned above, nearly all of the biomass was recovered. Physical and chemical properties of the up concentrated biomass are different. According to this model, the ideal RCF for the recovery pf the microalgae falls in the range of 6000 g which compared to the higher values, require less energy, while factors such as dry matter in the biomass and physiochemical properties shall also be considered.

### Example 6 - protein and lipid content by using different centrifugal forces in the centrifugation step

The effect of centrifugation at different relative centrifugal forces (RCF's) were evaluated on *Nannochloropsis salina* and *Chlorella sorokiniana.*

The microalgae cultures were before up-concentration by centrifugation harvested by using a silicon carbide membrane (300 mm x 0.1µm) at room temperature at 1.2±0.2 bar. The centrifugal forces varied from 1000-15000g. Centrifugation was done at constant temperature 20±1°C for 10 minutes. The protein and lipid content in samples was measured after harvest and after centrifugation with different centrifugal forces, 1000g. 4500g, 6300g, 10000g and 15000g.

The protein and lipid content in the samples is shown in figure 5. Figure 5 (a) shows lipid and protein contents (%-dry weight) of *Nannochloropsis salina* and figure (b) shows lipid and protein content (%-dry weight) of *Chlorella sorokiniana* for different RCF values.

As shown in figure 5, the content of protein increased in centrifuged samples, especially at higher RCF values, compared to the harvested sample. For *Chlorella sorokiniana,* variations in protein content were more apparent than *Nannochloropsis salina.* For the samples centrifuged at 15000 g, the protein content was measured to be 34±1.13 % dry-weight, which compared to the harvested sample (32±0.96 % dry-weight) was higher. However, only slightly higher. On the contrary, the lipid content decrease significantly when the relative centrifugal forces (RCF) increased for both *Chlorella sorokiniana* and *Nannochloropsis salina.* Variations in lipid content was higher for *Chlorella sorokiniana.* It is believed by the inventors of the present invention that the reason why the lipid content was lower at 15000g for *Chlorella sorokiana* than compared to the lipid content of *Nannochloropsis salina* at 15000g, is the difference of the average cell size of the two microalgae. The average cell size of *Chlorella sorokiniana* is 5-9 µm, while the average cell size for *Nannochloropsis salina* is 2-4µm. Thus, *Chlorella sorokiana* is bigger than *Nannochloropsis salina* and the degree of cell wall rupture caused by centrifugation was higher. Due to the rupture, some of the lipids (mostly membrane lipids) were liberated to the water phase, while extractability of the proteins are increased slightly for the same reason.

### Example 7 - fatty acid composition content by using different centrifugal forces in the centrifugation step

Variations in the fatty acid composition in a culture of *Nannochloropsis salina* with high content of polyunsaturated fatty acids were investigated.

As in example 5 and 6, the microalgae cultures were before up-concentration by centrifugation harvested by using a silicon carbide membrane (300 mm x 0.1µm) at room temperature at 1.2±0.2 bar. The centrifugal forces varied from 1000-15000g. Centrifugation was done at constant temperature 20±1°C for 10 minutes. The fatty acid content in samples was measured after harvest and after centrifugation with different centrifugal forces, 1000g, 4500g, 6300g, 10000g and 15000g.

The fatty acid content in the samples is shown in figure 6, where the results are presented as % of total fatty acids (n=2).

As shown in figure 6, when the RCF value increase, the contents of the polyunsaturated fatty acids decrease. In microalgae membrane lipids comprise polyunsaturated fatty acids such as eicosapentaenoic acid (EPA). EPA has very beneficial effects to health and rupture of cell wall results in loss of these compounds.

### Example 8 - content of pigment in relation to different centrifugal forces in the centrifugation step

Effects of centrifugation on the pigment composition in two microalgae species, *Nannochloropsis salina* and *Chlorella sorokiniana* were investigated.

As in example 5, 6, and 7 the microalgae cultures were before up-concentration by centrifugation harvested by using a silicon carbide membrane (300 mm x 0.1µm) at room temperature at 1.2±0.2 bar. The centrifugal forces varied from 1000-15000g. Centrifugation was done at constant temperature 20±1°C for 10 minutes. The pigment content in samples was measured after harvest and after centrifugation with different centrifugal forces, 1000g, 4500g, 6300g, 10000g and 15000g.

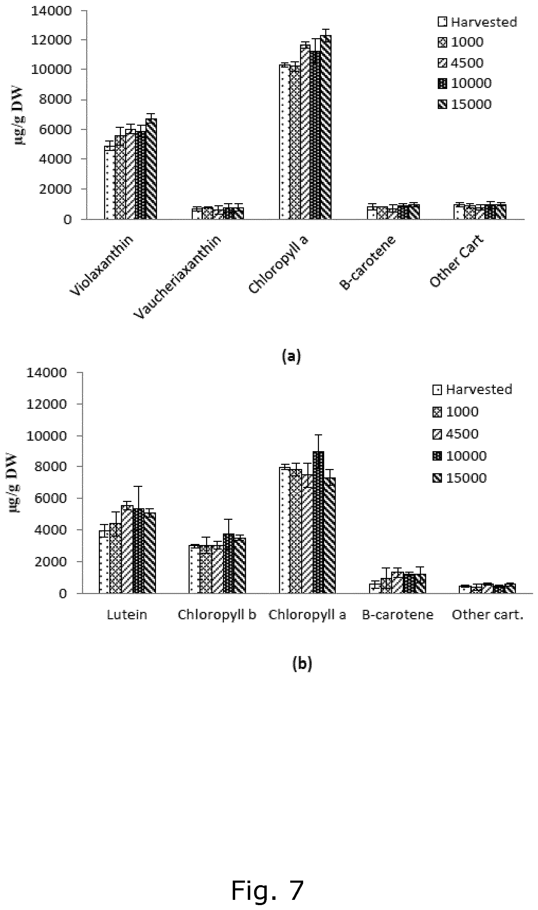

The pigment content in the samples is shown in figure 7. Figure 7 (a) shows the pigment composition (% dry-weight) in *Nannochloropsis salina* and figure 7 (b) shows the pigment composition (% dry-weight) in *Chlorella sorokiniana paste.* The pigments measured is violaxanthin, vaucheriaxnthin, chloropyll a, β-carotene and a pool of other carotenoids.

From figure 7, it is shown that all pigments, including chlorophylls, carotenes, and xanthophylls, higher concentrations were achieved in the samples which were centrifuged at higher RCF values. Higher concentrations of pigments is also due to cell wall rupture due to high centrifugal forces during centrifugation.

### Example 9 - leakage of lipids dependent on different centrifugal forces in the centrifugation step

Effects of centrifugation on the lipid leakage to the water phase for two microalgae species, *Nannochloropsis salina* and *Chlorella sorokiniana* were investigated.

As in example 5 to 8, the microalgae cultures were before up-concentration by centrifugation harvested by using a silicon carbide membrane (300 mm x 0.1µm) at room temperature at 1.2±0.2 bar. The centrifugal forces varied from 1000-15000g. Centrifugation was done at constant temperature 20±1°C for 10 minutes. The percentage of leakage (% dry weight of biomass) in samples was measured after harvest and after centrifugation with different centrifugal forces, 1000g. 4500g, 6300g, 10000g and 15000g.

Figure 8 shows the relationship between the percentage of leakage and relative centrifugal forces (1000 g, 4500 g, 6300 g, 10000 g and 15000 g), for *Chlorella sorokiniana* and *Nannochloropsis salina.*

It is shown in figure 8 that the degree of leakage increases as the centrifugal force under centrifugation increases. For Chlorella sorokiniana having a larger average cell size, the degree of the leakage is much higher, offering the fact that centrifugation of microalgae with larger cell sizes or thinner cell walls shall be done with lower centrifugal forces.

### Example 10 - viscosity dependent on different centrifugal forces in the centrifugation step

The viscosity of two microalgae species, *Nannochloropsis salina* and *Chlorella sorokiniana* were investigated when using different centrifugal forces under centrifugation.

As in example 5 to 9, the microalgae cultures were before up-concentration by centrifugation harvested by using a silicon carbide membrane (300 mm x 0.1µm) at room temperature at 1.2±0.2 bar. The centrifugal forces varied from 1000-15000g. Centrifugation was done at constant temperature 20±1°C for 10 minutes. The viscosity of samples was measured after harvest and after centrifugation with different centrifugal forces, 1000g. 4500g, 6300g, 10000g and 15000g.

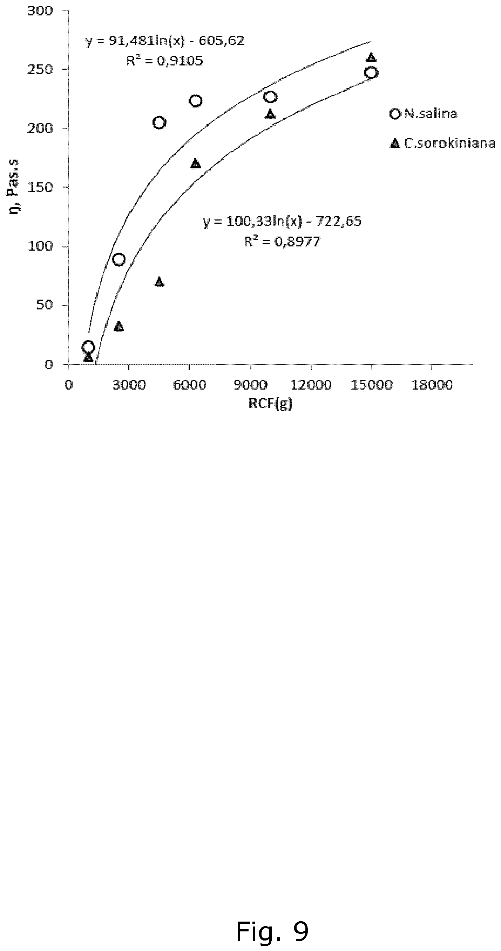

Figure 9 shows the relationship between the viscosity and relative centrifugal forces (1000 g, 4500 g, 6300 g, 10000 g and 15000 g), for *Chlorella sorokiniana* and *Nannochloropsis salina.* It is shown that the viscosity increases by increasing the centrigugal force. Both microalgae species *Nannochloropsis salina* and *Chlorella sorokiniana* showed the same pattern. Microalgae paste represents a pseudoplastic behaviour as the viscosity decreases upon shear stress and then stays constant over time.

### Example 11 - dry matter content in biomass dependent on different centrifugal forces in the centrifugation step

Concentration after centrifugation, in form of dry matter content, was investigated for three microalgae species, *Nannochloropsis salina, Chlorella sorokiniana* and *Chlorella vulgaris* when using different centrifugal forces under centrifugation.

As in example 5 to 10, the microalgae cultures were before up-concentration by centrifugation harvested by using a silicon carbide membrane (300 mm x 0.1µm) at room temperature at 1.2±0.2 bar. The centrifugal forces varied from 1000-15000g. Centrifugation was done at constant temperature 20±1°C for 10 minutes. The weight percentage of dry matter (DM%) of samples was measured after harvest and after centrifugation with different centrifugal forces, 1000g. 4500g, 6300g, 10000g and 15000g. Thus, all samples has been treated the same way before centrifugation and the only process parameter varying is the centrifugal force during centrifugation.

Figure 10 shows the relationship between dry matter content and relative centrifugal forces. Figure 10 shows that the concentration ratio highly varied between harvested microalgae species. The dry matter content in centrifuged pastes ranged from 12±2.0 % dry-weight to 38.3±1.0 % dry-weight for several microalgae investigated.

It can be seen in figure 10 that the initial concentration of dry matter for *Chlorella vulgaris* was higher than for *Nannochloropsis salina,* but the dry matter obtained at higher RCF values was apparently lower for *Chlorella vulgaris.* For all three types of microalgae, the dry matter content increased when the RCF increased. The dry matter content obtained was highest for Nannochloropsis salina and lowest for Chlorella vulgaris. Furthermore, figure 10 shows that for all three microalgae, the dry matter content increased the most at lower RCF values (1000 g to 10000 g), while the increase in dry matter when using even higher RCF's than 10000 g did lead to an increase in dry matter, but not as high as compared to lower RCF's.

### Example 12 - effect of heat treatment

In order to evaluate the effects of heat treatment on bioactive compounds in the microalgae biomass, a culture of the *Nannochlorosis salina* was, and afterwards harvested by membrane filtration with a silicon carbide membrane. After harvest, a portion of the harvested sample was pasteurized at 75°C for 15 seconds while another portion was not heat treated.

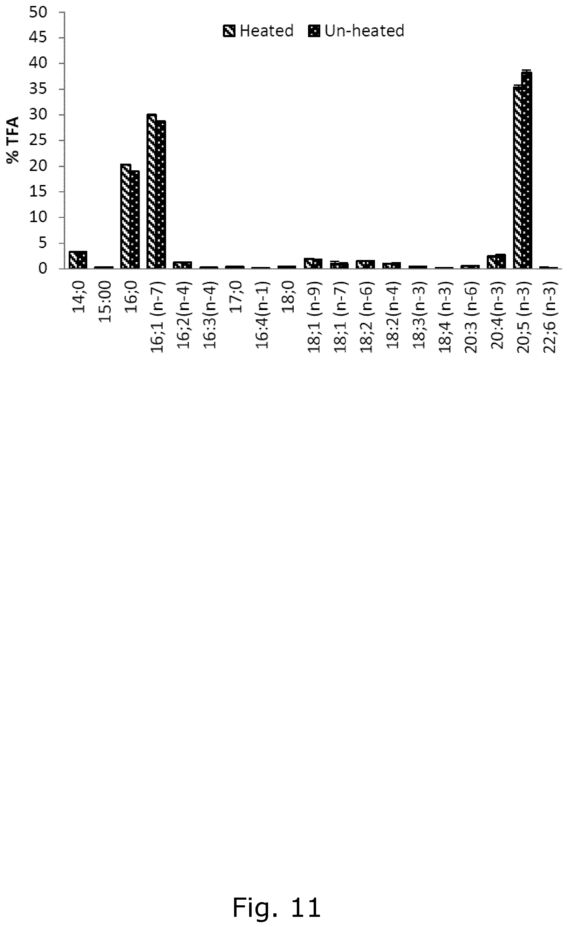

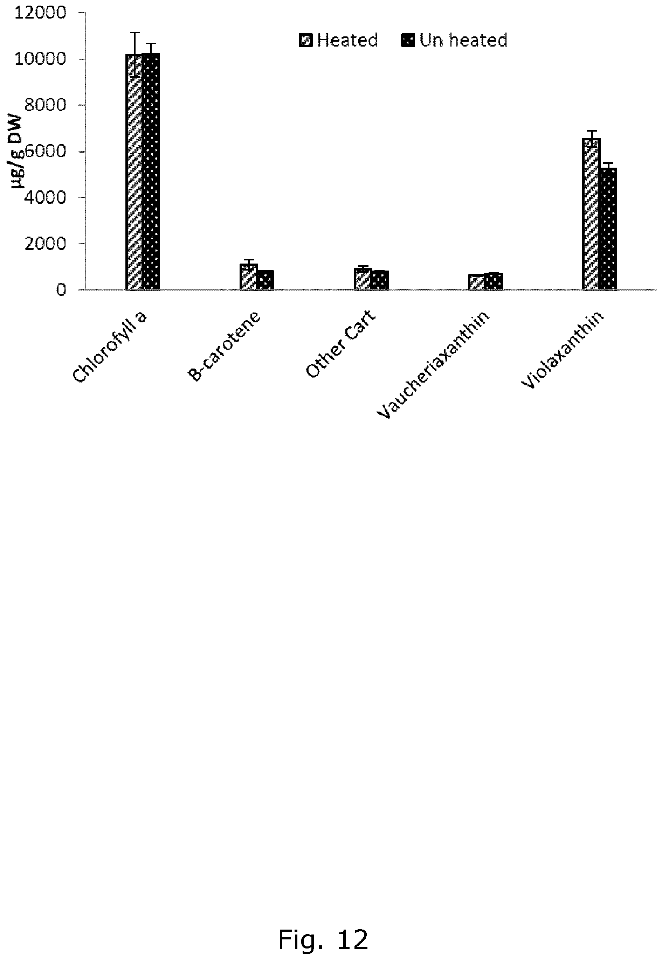

The content of fatty acids and pigments (Chlorophyll a, β-carotene, other carotenoids, Voucheriaxanthin and Violaxanthin) was measured. The result is shown in figure 11 and 12.

Figure 11 shows that only a 7.8% reduction of eicosapentaenoic acids (C20:5, n-3) is caused by the heat treatment. The reduction of eicosapentaenoic acid can be explained by the fact that this fatty acid is present in the cell wall membrane of microalgae.

Figure 12 shows that the content of the pigments was also affected by the heat treatment process. The content of Violaxanthin was surprisingly higher in the heated sample than in the unheated sample. This can be explained by with that the heat treatment could affect the cell wall and enhance the extractability of pigments. Figure 12 also shows that heat treatment do no alter the content of chlorophyll.

## Claims

1. A method for processing of microalgae biomass comprising the steps of:
a) harvesting microalgae by membrane filtration with a silicon carbide membrane, wherein a source of microalgae is passed through the silicon carbide membrane filter to obtain a retentate and a permeate
b) concentration by centrifugation of the retentate from step a) comprising a paste of microalgae biomass, and
c) drying the concentrated microalgae biomass from step b).

2. The method according to claim 1, wherein the centrifugal force under centrifugation is in the range of from 4000 g to 10000 g.

3. The method according to any of claims 1 or 2, wherein the source of microalgae is a suspension of microalgae.

4. The method according to any of the claims 1 to 3, wherein the method further comprises recirculation of the retentate from step a) to the source of microalgae and repeating step a) at least two times before concentration in step b).

5. The method according to any of the claims 1 to 4, wherein the method further comprises a step of pasteurization of the retentate obtained in step a) before concentration in step b).

6. The method according to claim 5, wherein the pasteurization is at a temperature of 60-120°C for 5-30 seconds.

7. The method according to any of the claims 1 to 7, wherein the pore size of the silicon carbide membrane is in the range of 0.05 to 0.5µm.

8. The method according to any of the claims 1 to 7, wherein the pressure during membrane filtration is in the range of 1 to 3 bar.

9. The method according to any of the claims 1 to 8, wherein the membrane filtration is dead-end membrane filtration or cross-flow membrane filtration.

10. The method according to any of claims 1 to 9, wherein a centrifuge used for centrifugation is a bowl centrifuge.

11. A microalgae biomass obtainable by the method according to any of claims 1 to 10.

12. Use of the microalgae biomass obtainable by the method according to any of the claims 1 to 10 in a feed.

13. Use of the microalgae biomass according to claim 12, wherein the feed is a feed for pigs, cows, poultry or fish.

14. Use of the microalgae biomass obtainable by the method according to any of the claims 1 to 10 in a food product.

15. Use of the microalgae biomass obtainable by the method according to claim 14, wherein the food product is selected from the group of dough, bread, buns, cake, pasta, cookies and energy bars.
